# EUROPÄISCHE PATENTSCHRIFT

(11) **EP 1 226 221 B1**
(45) Veröffentlichungstag und Bekanntmachung des Hinweises auf die Patenterteilung: **22.10.2003**
(21) Anmeldenummer: 00965840.2
(22) Anmeldetag: 07.09.2000
(51) Int. Cl.: C09K 11/02, F21K 2/00, A61K 41/00, A61N 5/06, H01J 61/46

(54) **LEUCHTSTOFFFOLIE**
FLUORESCENT FILM
FILM DE SUBSTANCE LUMINESCENTE

(30) Priorität: 20.09.1999 DE 19946125
(43) Veröffentlichungstag der Anmeldung: 31.07.2002
(73) Patentinhaber: PlasmaPhotonics GmbH, 12489 Berlin (DE)
(72) Erfinder: CONRADY, Jürgen, 13051 Berlin (DE)
(74) Vertreter: Effert, Bressel und Kollegen
(86) Internationale Anmeldenummer: DE0003155
(87) Internationale Veröffentlichungsnummer: WO01021728

(56) Entgegenhaltungen:
- EP-A- 0 518 718
- EP-A- 0 853 112
- PATENT ABSTRACTS OF JAPAN vol. 1997, no. 05, 30. Mai 1997 (1997-05-30) & JP 09 026511 A (FINE RUBBER KENKYUSHO:KK), 28. Januar 1997 (1997-01-28)

## Beschreibung

Die Erfindung betrifft eine Leuchtstofffolie, insbesondere zum Einsatz mit einer Niederdruckentladungslampe, ein Verfahren zur Herstellung der Leuchtstofffolie und eine Bestrahlungsanordnung mit der Leuchtstofffolie.

Lichtabsorption durch die Haut verursacht Gewebsänderungen durch Beeinflussung des neuronalen, des lymphatischen, des vaskulären und des Immunsystems. Hierdurch kommt es zu analgetischen, antientzündlichen, antiödematösen Wirkungen und zu einer Stimulation von Wundheilungen. Unter einer Einstrahlung von rotem Licht (660 nm, 2,4-4 J/cm²) wurde eine erhebliche Zunahme von Fibroblasten aus Narbengewebe festgestellt (Webb, C.; M.Dyson et al, Lasers In Surgery And Medicine, 22(5), S.294-30, (1998)). Bei Bestrahlung von peripheren Lymphozyten mit einem He-Ne-Laser mit Bestrahlungsdosen zwischen 28 und 112 J/m² kam es zu einer Zunahme der RNA-Synthese nach Stimulation der Lymphozyten durch Cytohämagglutinin (Smol'yaninova, N.K., T.I.Karu, et al. Biomedical Science, 2(2), S. 121-126, (1991)). Bei Knochenverletzungen wurde nach He-Ne-Laser Bestrahlung eine Verdoppelung des Kalziumeinbaus an der Verletzungsstelle beobachtet (Yaacoby, T., L. Maltz, et al. Calcified Tissue International 59(4), S. 297-300, (1996)). Verschiedene chronische Gelenkerkrankungen wie Gonarthrosis, LWS-Arthrose und Algodystrophie bei halbseitig gelähmten Patienten bei Schlaganfall wurden positive Wirkungen einer He-Ne-Laser-Bestrahlung bei über 400 Patienten festgestellt (Giavelli, S., G. Fava, et al. Radiologia Medica, 95(4), S. 303-309, (1998)). Als mögliche Ursache für die positiven Effekte werden die Freisetzung von Interleukin-1-alpha und Interleukin-8 diskutiert (Yu. H.S., K.L. Chang, et al. Journal Of Investigative Dermatology 107(4), S 593-596, (1996)). Unter einer Bestrahlung von 1,5 J/cm² kam es zu einer konzentrationsabhängigen Simulation sowohl der Interleukin-1-alpha-Produktion als auch der entsprechenden mRNA-Expression. Da diese Cytokine sowohl die Beweglichkeit als auch die Proliferation von Keratinocyten stimulieren, ist eine direkte Förderung der Wundheilung durch diese Mechanismen wahrscheinlich. Darüber hinaus werden Modelle des photonischen zellulären Energietransfers in Bezug auf die Atmungskette diskutiert (Wilden, L and R. Karthein, Journal Of Clinical Laser Medidne And Surgery 16(3), S 159 -165, (1998)). Die biochemischen Modelle des zellulären Energietransfers betrachten lediglich den klassischen korpuskularen Aspekt von Elektronen als verantwortliche Energieüberträger und ignorieren den Welle-Teilchen-Dualismus von Elektronen beim Energieeintrag. Licht des roten und des nahen Infrarotbereiches korrespondiert gut mit charakteristischen Energieebenen und Absorptionsraten wichtiger Bestandteile der Atmungskette. Hierdurch kommt es beispielsweise zu einem Anstieg der mitochondrialen Adenosin-Triphosphat-Produktion. Aufgrund dieser Interaktion werden Wechselwirkungen im roten und nahen IR-Bereich erklärbar.

Photobiologische Wirkungen im Nicht-UV-Bereich auf Grundlage einer Wechselwirkung zwischen endogenen oder exogenen Chromophoren in der Haut gewinnen zunehmend an Bedeutung, da mit Hilfe geeigneter Strahlungsquellen therapeutische Wirkungen bei bestimmten entzündlichen Hauterkrankungen und beispielsweise Wundheilungsstörugnen bei Diabetes Mellitus beeinflußbar sind.

Aufgrund ihres meist besseren Wirkungsgrades gegenüber Hochdrucklampen oder Temperaturstrahlern finden Niederdruckentladungslampen in vielen Gebieten der Technik verstärkt Anwendung, insbesondere wenn hohe Lichtenergieausbeuten benötigt werden. Je nach Anwendungsgebiet sind ein- oder zweiseitig gesockelte Niederdruckentladungslampen bekannt. Weiter können diese mit oder ohne Leuchtstoff und mit verschiedenen Gasen ausgebildet sein. Allen Ausführungsformen ist jedoch gemeinsam, daß die Lichtenergiedichte mit kleiner werdendem Hüllkörperdurchmesser ansteigt.

Entsprechend einer Modellrechnung entspricht die Lichtenergiedichte in etwa einem Viertel des Quotienten aus Säulenleistung und Projektionsfläche. Dies bedeutet, daß der theoretische Maximalwert einer 38 mm Niederdruckentladungslampe bei ca. 45 mW/cm² liegt. Bei einer 26 mm Niederdruckentladungslampe steigt die Lichtenergiedichte auf ca. 50 mW/cm². Für die Lampendurchmesser 16, 12 und 8 mm ergeben sich theoretisch Lichtenergiedichten von 100, 125 und 170 mW/cm². Die erhöhte Leuchtdichte kleiner Strahler wird beispielsweise bei der Konstruktion von Kompaktlampen ausgenutzt, die z.B. 12 mm Wanddurchmesser aufweisen. Für Effektbeleuchtungen sind seit einigen Jahren 8 mm Leuchtstoffröhren im Einsatz. Diese übertreffen die Kompaktlampen an Leuchtdichte, jedoch betragen die längsten lieferbaren Längen nur ca. 30 cm.

Die Verkleinerung der Lampengeometrie hat jedoch trotz der Erhöhung der Lichtleistung gravierende Nachteile. Um strahlende Flächen zu erzeugen, benötigt man eine Vielzahl von Lampen mit ebenso vielen teuren Vorschaltgeräten. Der Verlängerung der Lampen sind plasmaphysikalische Grenzen gesetzt, da die erforderlichen großen Zündspannungen für große Längen einen erheblichen Aufwand bedeuten. Hinzu kommen die Fertigungskosten selbst, d.h. das Beschlämmen, Pumpen, und Sockeln jeder einzelnen Leuchtstoffröhre.

Zur Flächenbelichtung werden daher meist Niederdruckentladungslampen mit externen oder internen Reflektoren verwendet, mit denen beispielsweise bei 100 W Bestrahlungstärke zwischen 22-28 mW/cm² Lichtenergiedichte erreichbar sind. Allerdings sind die tatsächlich erreichbaren Lichtenergierdichten erheblich unter den theoretisch erreichbaren.

Grundsätzliches Problem der klassischen Niederdruckentladungslampen mit fluoreszierendem Leuchtstoff und elektronenemittierenden Elektroden ist die begrenzte Nutzungszeit, vor allem bei sehr hohen Lampenleistungen.

Hauptursache hierfür ist, daß Reaktionsbestandteile des Elektrodenabbrandes mit der Leuchtstoffschicht chemisch reagieren, was zu einem "Alterungsprozeß" führt. Ein weiteres Problem ist, daß die Reaktionsbestandteile des Elektrodenabbrandes und des Quecksilberdampfes mit alkalischen Verbindungen der Glasröhre zu verschiedenen Amalgamen reagieren. Dies führt zu einer Schwärzung der Röhre , einer beschleunigten Verminderung der Lichtleistung und einer zum Teil dramatischen Verkürzung der Lampenlebensdauer. Da bereits die Lebensdauer aufgrund des Alterungsprozesses der Leuchtstoffschicht stark begrenzt ist, hat sich bisher der Einsatz teurer alkalifreier Quarzgläser nicht gelohnt. Für medizinische Hochleistungsstrahler kann die Nutzungsdauer z.B. nur 48 Stunden betragen.

Versuche, den Leuchtstoff auf der Außenseite der Niederdruckentladungslampe aufzubringen, waren nicht erfolgreich, da der Auftrag von Leuchtstoff in einer nicht-inerten Atmosphäre zu einer photochemischen oxidativen Zersetzung des hygroskopischen Leuchtstoffs führt.

Aus der US- 5,717,282 ist eine Braunsche Röhre für die Monitorproduktion bekannt, wobei auf der Außenseite des Monitors ein silikathaltiger Lack mit Leuchtstoffen aufgebracht ist, der im Sol- Gel hergestellt wird. Die Dicke dieser Phosphorschicht ist auf ca. 0,5µm begrenzt, da es ansonsten wegen der großen Schrumpfung des anorganischen Netzes zu Rissen kommt. Derartige Schichtdicken sind jedoch zu dünn und thermisch nicht ausreichend stabil für den Einsatz in einer Niederdruckentladungslampe bei höheren Leistungen.

Aus der US- 5,731,658 ist eine Flüssigkristallanzeige bekannt, auf deren inneren Begrenzungswänden eine Phosphorschicht aufgebracht wird. Die Phosphorschicht besteht aus einem UV-transparenten Trägermaterial und Phosphor. Als Trägermaterial wird Siliziumoxid oder Organo-Silicate, insbesondere Ethyl-, Methyl- oder Isopropyl-Silicat vorgeschlagen. Auch die hiermit erreichbaren Schichtdicken sind zu gering, um ausreichend Leuchtstoff für eine Niederdruckentladungslampe einzubetten.

Der Erfindung liegt daher das technische Problem zugrunde, eine Leuchtstofffolie zu schaffen, die bei guter thermischer Stabilität in ausreichender Dicke herstellbar ist, so daß diese zum Einsatz für Niederdruckentladungslampen geeignet ist. Ein weiteres technisches Problem liegt in der Schaffung einer flexiblen Bestrahlungsanordnung, die für die verschiedensten Anwendungsgebiete einsetzbar ist. Ein weiteres technisches Problem liegt in der Schaffung eines Herstellungsverfahrens für eine Leuchtstofffolie.

Die Lösung des technischen Problems ergibt sich durch die Gegenstände mit den Merkmalen der Patentansprüche 1, 11 und 23. Weitere vorteilhafte Ausgestaltungen der Erfindung ergeben sich aus den Unteransprüchen.

Durch die Ausbildung der Leuchtstofffolie als Silikonelastomere, in das die Leuchtstoffpartikel eingebettet sind, können einerseits Folien ausreichender Dicke mit einer genügend hohen Leuchtstoffkonzentration hergestellt werden. Des weiteren sind die Leuchtstoffpartikel luftdicht und wasserfrei in dem Silikonelastomere vernetzt, so daß diese keinem Alterungsprozeß ausgesetzt sind. Silikonelastomere sind UVC- durchlässig und weisen gegenüber alternativen UVC- durchlässigen Trägermaterialien erhebliche Vorteile auf. Saphir und Quarz sind zwar UVC- durchlässig, jedoch ist es aus leuchtstoffchemischen Gründen nicht möglich, anorganische Leuchtstoffe als Dotierung in Quarzfenster einzusetzen. Eine Saphirdotierung scheidet wegen der extremen Schmelztemperaturen von vornherein aus. Andere Kunststoffe wie beispielsweise Acrylate, transparentes PVC oder Teflon sind nicht ausreichend thermostabil. Die Silikonelastomere sind dagegen bis 250°C stabil und benötigen keine Weichmacher oder andere flüchtige Substanzen, die abdampfen könnten. Aufgrund der verlängerten Lebensdauer des Leuchtstoffes, dadurch daß Leuchtstoff außerhalb des Ladungsgefäßes angeordnet werden kann und somit keine Reaktion mit dem Elektrodenabbrand auftreten kann, ist auch der Einsatz alkalifreier Quarzgläser akzeptabel, was die Lebensdauer und Qualität der Niederdruckentladungslampe weiter erhöht.

In einer bevorzugten Ausführungsform ist das Silikonelastomere durch ein Verfahren herstellbar, bei dem ein Hydroxylpolydiorganosiloxan mit einem Organohydrogensiloxan unter Zuführung der Leuchtstoffpartikel in kristalliner Form vorliegen können. Mittels eines Platinkatalysators ist dann bei Raumtemperatur eine chemische Reaktion erzeugbar, die zu einer vollständigen Vernetzung führt, wobei aufgrund der geringen Prozeßtempereraturen die Leuchtstoffpartikel nicht belastete werden.

Als besonders geeignet hat sich Hydroxylpolydiorganosiloxan aus verschiedenen Polymeren mit einer Mindestviskosität von 1000 Centipoise bei Raumtemperatur erwiesen, wobei das Hydroxyldiorganosiloxan vorzugsweise als Hyxdroxylpolydimethylsiloxan, dessen Copolymeren, Phenylmethylsiloxan und / oder Polymethyl -3, 3, 3- Trifluoropropylsiloxan ausgebildet ist.

Das Organohydrogensiloxan ist vorzugsweise als Silikon mit mindestens 2-silikongebundenen Wasserstoffatomen pro Molekül ausgebildet, insbesondere aus Homopolymeren, Copolymeren oder deren Mischungen.

Der Platinkatalysator kann aus einem Platinsalz, insbesondere Platinchlorid oder Chlorplatinsäure bestehen, wobei letztere vorzugsweise als Hexahydrat oder in wasserfreier Form verwendet wird.

Die Dicke der Leuchtstofffolie liegt vorzugsweise im Bereich zwischen 10- 800 µm, wobei die Flächendichte dabei zwischen 1 - 20 mg/cm² beträgt. Besonders vorteilhaft erscheinen Dicken zwischen 100- 600 µm mit einer Flächendichte zwischen 3- 6 mg/cm². Die Korngröße der Leuchtstoffpartikel liegt vorzugsweise zwiscen 5 - 15 µm.

Vorzugsweise ist die Leuchtstofffolie auf der Außenseite des Hilfskörpers angebracht.

Durch die Anordnung der Leuchtstofffolie außerhalb des Entladungsraumes kann eine sehr flexibel handhabbare Bestrahlungsanordnung aufgebaut werden. Zum einem ist die Lebensdauer der Bestrahlungsanordnung nur noch durch die Niederdruckentladungslampe selbst, insbesondere von deren Elektroden abhängig, da die Leuchtstofffolien selbst jederzeit einfach austauschbar sind. Dies ermöglicht darüber hinaus eine sehr einfache Bestückung mit verschieden dotierten Leuchtstofffolien, so daß sich mit einer Bestrahlungsanordnung unterschiedliche Spektralbereiche und Bestrahlungsstärken einstellen lassen.

In einer bevorzugten Ausführungsform ist in dem Hüllkörper ein Verdrängungskörper angeordnet, so daß sich zwischen Hüllkörper und Verdrängungskörper Kanäle ausbilden, wodurch die Niederdruckentladungslampe sehr lang ausgeführt werden kann, ohne daß sehr große Zündspannungen benötigt werden, da immer noch ein ausreichend großes Plasmavolumen verbleibt. Andererseits steigt die emittierte Lichtenergiedichte in den Kanälen zwischen dem Hüllkörper und dem Verdrängungszylinder an, da der Kanal wie eine Niederdruckentladungslampe mit kleinem Durchmesser wirkt. Sind Hüllkörper und Verdrängungskörper als Zylinder ausgebildet, so bildet sich ein Zylindermantel als Kanal aus, den man anschaulich als viele radial zueinander angeordnete Niederdruckentladungslampen mit kleinem Durchmesser auffassen kann.

In einer bevorzugten Ausführungsform ist der Verdrängungskörper als geschlossener Hohlkörper ausgebildet, was insbesondere hinsichtlich des Gewichts von Vorteil ist.

Auf der Außenseite des Verdrängungskörpers kann auch eine Reflektorschicht aufgebracht werden oder aber der Verdrängungskörper kann aus einem für die emittierte Strahlung der Gasatome transparentem Material bestehen. Darüber hinaus ist auch eine Kombination der Maßnahmen möglich.

Zur Herstellung von Niederdruckentladungslampen mit unterschiedlichen Lichtenergiedichten kann eine Befestigungsvorrichtung zur Aufnahme unterschiedlichere Verdrängungskörper verwendet werden. Je nachdem was für eine Lichtenergiedichte gewünscht wird, wird dann bei der Herstellung ein Verdrängungskörper unterschiedlichen Durchmessers eingesetzt.

In einer weiteren bevorzugten Ausüfhrungsform ist der Verdrängungskörper unregelmäßig ausgeformt, sodass der Kanal zwischen Hüllkörper und Verdrängungskörper entlang der Längsrichtung unterschiedliche Breiten aufweist.

Bei bestimmten Anwendungen ist es wünschenswert, keine gleichmäßige Lichtenergiedichte über die gesamte Bestrahlungsfläche zu erhalten. Beispielsweise möchte man bei Sonnenliegen eine verstärkte Strahlung nur im Kopfbereich. Diese läßt sich leicht dadurch erreichen, daß beispielsweise sich der Verdrängungskörper nur entlang des Kopfbereiches erstreckt oder aber der Verdrängungskörper in Längsrichtung unterschiedliche Durchmesser aufweist. Eine weitere Möglichkeit besteht darin, den Verdrängungskörper an den gewünschten Stellen mit einer Reflektorschicht zu beschichten.

Durch die Möglichkeit verschiedene leinwandähnliche Bestrahlungsfolien mit unterschiedlichen Leuchtstoffen an ein und derselben Lichtquelle zu betreiben, entsteht ein sehr vielseitiges Therapie- und Bestrahlungssystem. Der behandelnde Arzt kann ähnlich dem Einsetzen eines großen optischen Filters in sehr kurzer Zeit, d.h. in einer Minute durch Wechseln der Silikonmodule einen anderen Patienten behandeln bzw., gealterte Silikonmodule ersetzen.

In einer weiteren bevorzugten Ausführungsform wird die zu behandelnde Körperpartie mit der erfindungsgemäßen Leuchtstofffolie verbandähnlich umwickelt.

Die Erfindung wird nachfolgend anhand eines bevorzugten Ausführungsbeispieles näher erläutert. Die Fig. zeigen:
- Fig. 1: eine schematische Draufsicht auf eine Bestrahlungsanordnung,
- Fig. 2: eine schematische Teildraufsicht auf eine Niederdruckentladungslampe Niederdruckentladungslampe
- Fig. 3: Spektren verschiedener Leuchtstofffolien,
- Fig. 4: ein Diagramm der Intensitäten über der Foliendicke,
- Fig. 5: ein Diagramm der Intensitäten über der Flächendichte der Leuchtstoffpartikel und
- Fig. 6: spektrale Absorptionsverläufe einer Leuchtstofffolie im Zeitverlauf.

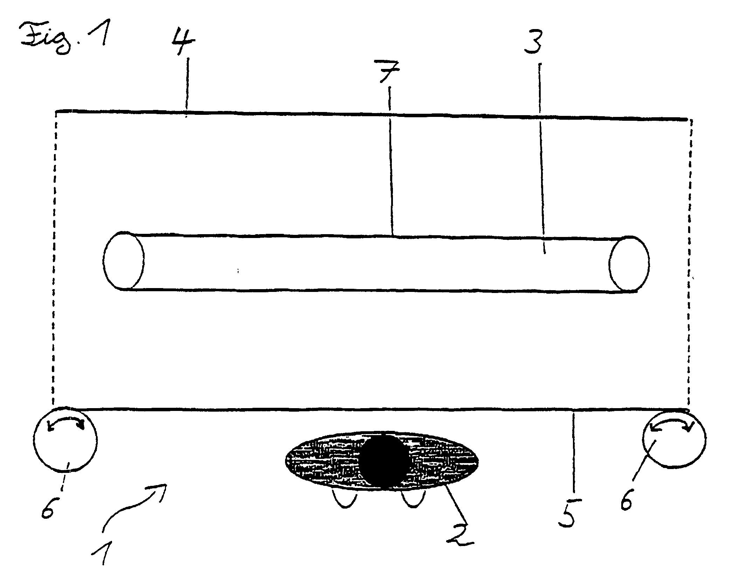

In der Fig. 1 ist eine schematische Draufsicht auf eine Bestrahlungsanordnung 1 zur kosmetischen und/oder therapeutischen Behandlung eines Patienten 2 dargestellt. Die Bestrahlungsanordnung 1 umfaßt mindestens eine Niederdruckentladungslampe 3, einen Reflektorschirm 4 und eine Leuchtstofffolie 5, die mittels Walzen 6 auf- und abwickelbar gelagert ist. Die dargestellten Abstände zwischen der Niederdruckentladungslampe 3 und dem Reflektorschirm 4 bzw. der Leuchtstofffolie 5 sind dabei nicht maßstäblich. Die im Entladungsprogramm der Niederdruckentladungslampe 3 erzeugte UV-Strahlung tritt isotrop aus dem UV- durchlässigen Hüllrohr 7 der Niederdruckentladungslampe 3 aus und trifft teilweise direkt auf die Leuchtstofffolie 5. Ein anderer Anteil der Strahlung trifft auf die Reflektorschicht 4 und wird von dort teilweise auf die Leuchtstofffolie 5 reflektiert. Die auf die Leuchtstofffolie 5 auftreffende UV- Strahlung regt teilweise die in der Leuchtstofffolie 5 eingebetteten Leuchtstoffpartikel an, die dann im gewünschten Spektralbereich emittieren und den Patienten bestrahlen. Mittels der Walzen 6, auf die ein Teil der Leuchtstofffolie 5 aufgewickelt ist, lassen sich verschiedene Arten von Bestrahlungsanordnungen 1 realisieren.

Im einfachsten Fall erstrecken sich die Walzen 6 über die volle Höhe der Bestrahlungsanordnung 1, auf die eine einheitlich dotierte Leuchtstofffolie 5 aufgewickelt ist. Falls dann der sich im abgewickelten Bereich befindlichen Leuchtstoff gealtert sein sollte, so wird dieser Bereich aufgewickelt und ein entsprechend unverbrauchter Teil der Leuchtstofffolie 5 abgewickelt. Des weiteren ist es auch möglich, verschieden dotierte Leuchtstofffolien 5 zu verwenden, so daß je nach gewünschter Bestrahlungstherapie ein bestimmter Bereich der Leuchtstofffolie 5 mit der passenden Dotierung abgewickelt wird. Darüber hinaus ist es möglich über die Höhe verschiedene Walzen 6 vorzusehen, so daß die zuvor beschriebene Variation zusätzlich für verschiedene Körperpartien vornehmbar ist.

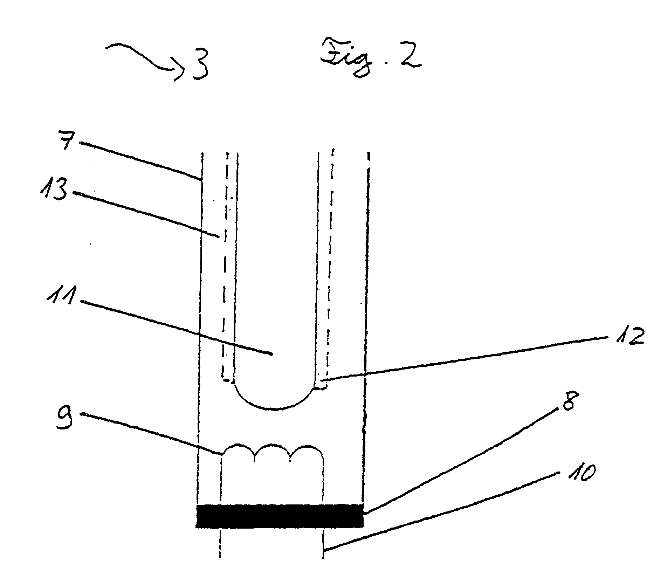

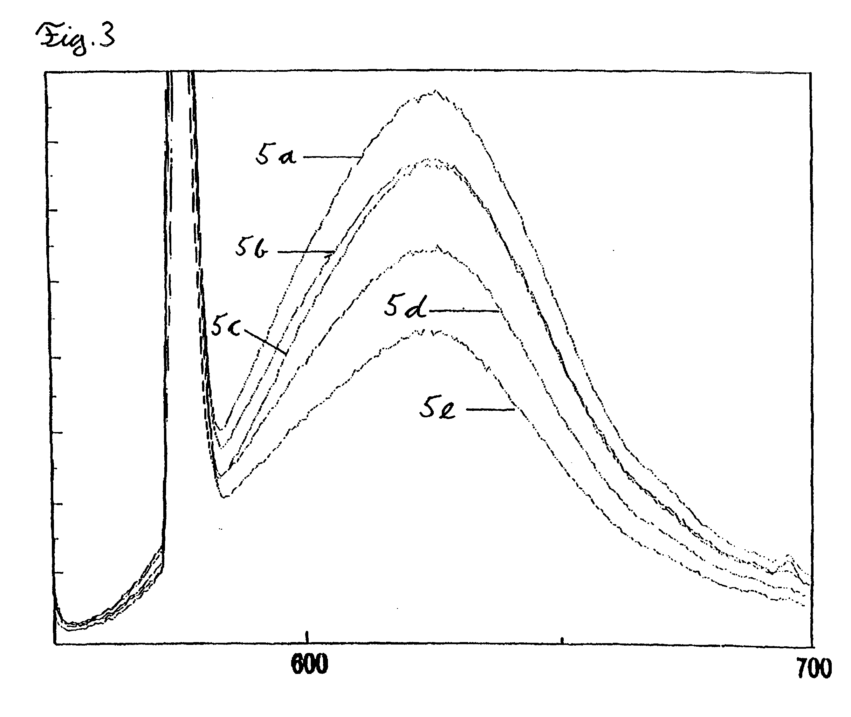

In der Fig. 2 ist eine schematische Teildraufsicht auf eine bevorzugte Ausführungsform einer Niederdruckentladungslampe 3 dargestellt. Die Niederdruckentladungslampe 3 umfaßt einen Hüllkörper 7, ein den Hüllkörper 7 hermetisch abschließender Sockel 8, eine Glühwendel 9 mit durch den Sockel 8 geführten Kontakten 10 und einen als Hohlkörper ausgebildeten Verdrängungskörper 11. Der Verdrängungskörper 11 ist rotationssymmetrisch zum Hüllkörper 7 angeordnet und etwas von der Glühwendel 9 beabstandet. Auf der Außenseite des Verdrängungskörpers 11 ist eine reflektierende Beschichtung 12 aufgebracht. Zwischen dem Hüllkörper 7 und dem Verdrängungskörper 11 bildet sich ein rotationssymmetrischer Kanal 13 mit dem Niederdruckplasma aus, wobei als Füllstoff vorzugsweise Quecksilber mit Argon verwendet wird. Über die Glühwendel 9 werden mittels thermischer Emission Elektronen emittiert und durch ein äußeres elektrisches Feld beschleunigt. Dabei kommt es zu einer Wechselwirkung mit den Quecksilberatomen im Kanal 13. Durch die Wechselwirkung werden die Elektronen des Quecksilbers angeregt, die dann die aufgenommene Energie mittels spontaner Emission von Photonen wieder abgeben. Die dadurch entstehende UV- Strahlung verläßt dann direkt oder nach Reflexion an der Beschichtung 12 den Hüllkörper 7 und regt die Leuchtstoffpartikel in der außerhalb der Niederdruckentladungslampe 3 angeordneten Leuchtstofffolie an. In der Fig. 3 sind die Intensitäten verschiedener Leuchtstofffolien mit unterschiedlicher Foliendicke und unterschiedlicher Dotierungskonzentration für einen Leuchtstoff LS 635 dargestellt. Die Leuchtstofffolien 5a- e weisen dabei die folgenden Parameter auf:

| Folie | Foliendicke (mm) | Dotierung (g/cm³) | Flächendichte der Leuchtstoffpartikel in mg/cm² |
|---|---|---|---|
| 5 a | 0,2 | 0,2 | 4 |
| 5 b | 0,55 | 0,1 | 5,5 |
| 5 c | 0,6 | 0,2 | 12 |
| 5 d | 0,25 | 0,5 | 12,5 |
| 5 e | 0,65 | 0,3 | 19,5 |

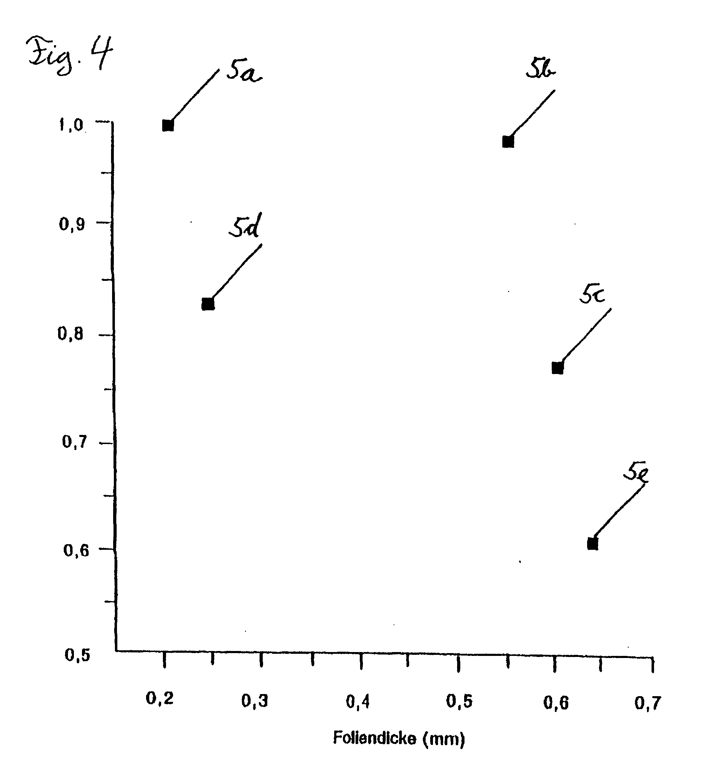

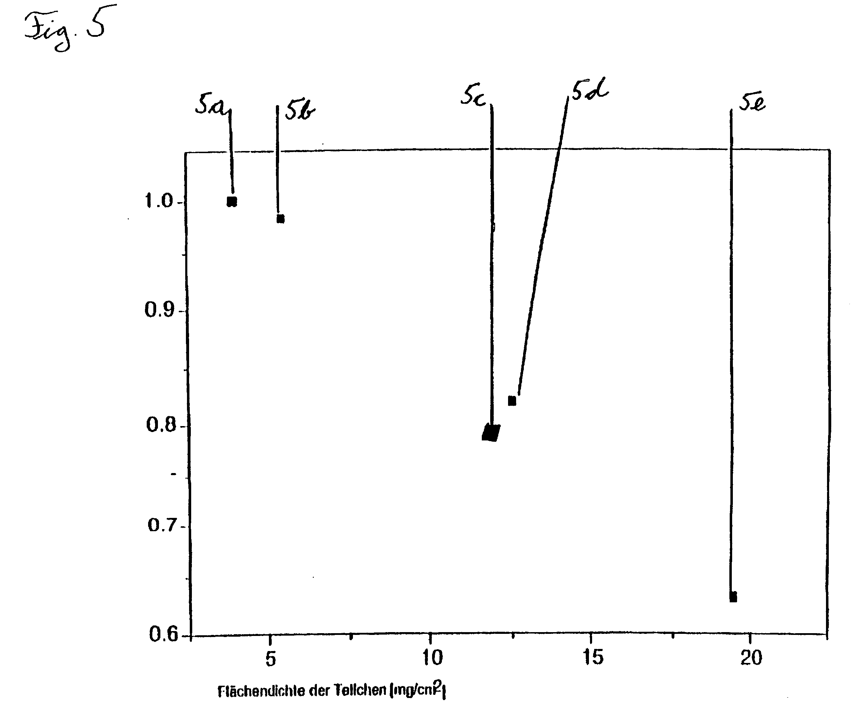

In der Fig. 4 und 5 sind die Leuchtstofffolien 5a- e mit einer normierten Intensität über der Foliendicke bzw. der Flächendichte der Leuchtstoffpartikel dargestellt. Wie man insbesondere Fig. 5 entnehmen kann, ergeben sich hohe Intensitäten im Bereich von 4- 6 mg/cm² Flächendichte der Leuchtstoffpartikel. Des weiteren ist beispielsweise anhand der Leuchtstofffolie 5e erkennbar, daß besonders dicke Folien mit einer hohen Dotierung nicht zu großen Intensitäten führen, was vermutlich auf Abschattungseffekte und Selbstanregung zurückzuführen ist. Die vorliegenden Meßergebnisse legen den Schluß nahe, daß vermutlich leuchtstoffabhängig jeweils bezüglich Foliendicke und Flächendichte ein Optimum existiert, die vermutlich empirisch ermittelt werden müssen. Allerdings legt Fig. 5 nahe, daß der entscheidende Parameter die Flächendichte der Leuchtstoffpartikel ist, da sich die Folien 5a und 5b bzw. 5c und 5d sich trotz erheblicher Abweichungen in der Dicke nahezu gleich verhalten.
Prinzipiell scheinen daher dünne Folien geeigneter zu sein, da diese erheblich weniger Material für die gleiche Intensität erfordern, jedoch muß noch deren Temperaturbeständigkeit und Lebensdauer im Vergleich zu dickeren Folien näher untersucht werden.

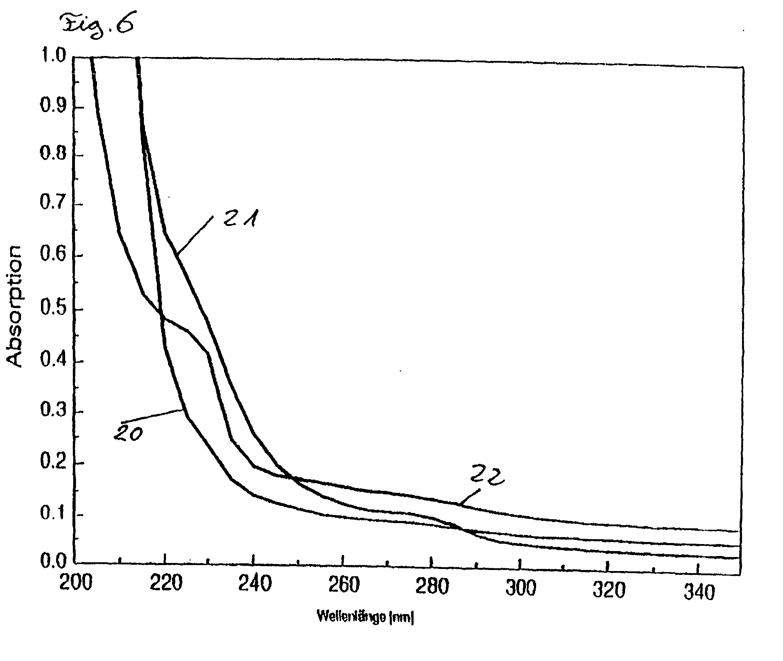

In der Fig. 6 ist der spektrale UV- Absorptionsverlauf 20 einer 530 µm dicken Leuchtstofffolie dargestellt. Des weiteren ist der UV- Absorptionsverlauf 21 dieser Folie nach 5 Tagen Dauerbelastung durch eine 54 W- UV- Lampe bei 60°C bei 2 cm Abstand und der UV- Absorptionsverlauf 22 nach 7 Tagen Dauerbelastung durch eine 54 W- UV- Lampe bei 60°C, wobei die Folie direkt auf dem Hüllrohr auflag, dargestellt. Diese Verläufe stellen eindrucksvoll die lange Lebensdauer der Folie dar, deren UV- Absorptionsverlauf auch bei Dauerbelastung nahezu unverändert ist.

## Patentansprüche

1. Leuchtstofffolie, insbesondere zum Einsatz mit einer Niederdruckentladungslampe (3), wobei die Leuchtstofffolie (5) als Silikonelastomere ausgebildet ist, in das die Leuchtstoffpartikel eingebetfet sind,
**dadurch gekennzeichnet, daß**
das Silikonelastomere durch folgendes Verfahren herstellbar ist:
a) Mischen eines Hydroxylpolydiorganosiloxans mit einem Organohydrogensiloxan,
b) Zuführen von Leuchtstoffpartikeln und
c) Erzeugen einer chemischen Reaktion mittels eines Platinkatalysators bei Raumtemperatur.

2. Leuchtstofffolie nach Anspruch 1, **dadurch gekennzeichnet, daß** das Hydroxylpolydiorganosiloxan aus verschiedenen Polymeren mit einer Mindestviskosität von 1000 Centipoise bei 25°C besteht.

3. Leuchtstofffolie nach Anspruch 2, **dadurch gekennzeichnet, daß** das Hydroxylpolydiorganosiloxan als Hydroxylpolydimethylsiloxan, dessen Copolymeren, Phenylmethylsiloxan und / oder Polymethyl -3, 3, 3-Trifluoropropylsiloxan ausgebildet ist.

4. Leuchtstofffolie nach Anspruch 2 oder 3, **dadurch gekennzeichnet, daß** das Organohydrogensiloxan als Silikon mit mindestens 2-silikongebundenen Wasserstoffatomen pro Molekül ausgebildet ist.

5. Leuchtstofffolie nach Anspruch 4, **dadurch gekennzeichnet, daß** das Organohydrogensiloxan aus Homopolymeren, Copolymeren oder deren Mischungen besteht.

6. Leuchtstofffolie nach einem der vorangegangenen Ansprüche, **dadurch gekennzeichnet, daß** der Platinkatalysator aus Platinchlorid, Platinsalzen oder Chlorplatinsäure besteht.

7. Leuohtstofffolie nach Anspruch 6, **dadurch gekennzeichnet, daß** die Chlorplatinsäure als Hexahydrat oder in wasserfreier Form vorliegt.

8. Leuchtstofffolie nach einem der vorangegangenen Ansprüche, **dadurch gekennzeichnet, daß** die Leuchtstofffolie (5) zwischen 10 bis 800 µm dick ist

9. Leuchtstofffolie nach einem der vorangegangenen Ansprüche, **dadurch gekennzeichnet, daß** die Flächendichte der Leuchtstoffpartikel 1-20 mg/cm² beträgt.

10. Leuchtstofffolie nach einem der vorangegangenen Ansprüche, **dadurch gekennzeichnet, daß** die Korngröße der Leuchtstoffpartikel zwischen 5-15 µm beträgt.

11. Bestrahlungsanordnung, umfassend eine Niederdruckentladungslampe, mit einem UVC- durchlässigen Hüllkörper, in dem von außen kontaktierbare Elektroden hineinragen, und eine Leuchtstoffschicht,
**dadurch gekennzeichnet, daß**
die Leuchtstoffschicht als Leuchtstofffolie (5) nach Anspruch 1 ausgebildet ist, in die Leuchtstoffpartikel eingebettet sind.

12. Bestrahlungsanordnung nach Anspruch 11, **dadurch gekennzeichnet, daß** die Leuchtstofffolie (5) auf der Außenseite des Hüllkörpers (7) angebracht ist.

13. Bestrahlungsanordnung nach Anspruch 12, **dadurch gekennzeichnet, daß** auf dem Hüllkörper (7) verschieden dotierte Leuchtstofffolien (5) aufgebracht sind.

14. Bestrahlungsanordnung nach einem der Ansprüche 11 bis 13, **dadurch gekennzeichnet, daß** in dem Hüllkörper (7) ein Verdrängungskörper (11) angeordnet ist, so daß sich zwischen Hüllkörper (7) und Verdrängungskörper (11) Kanäle (13) ausbilden.

15. Bestrahlungsanordnung nach Anspruch 14, **dadurch gekennzeichnet, daß** der Verdrängungskörper (11) als geschlossener Hohlkörper ausgebildet ist.

16. Bestrahlungsanordnung nach einem der Ansprüche 14 oder 15, **dadurch gekennzeichnet, daß** mindestens teilweise auf der Außenseite des Verdrängungskörpers (11) eine Reflektorschicht (12) aufgebracht ist.

17. Bestrahlungsanordnung nach einem der Ansprüche 14 bis 16, **dadurch gekennzeichnet, daß** der Verdrängungskörper (11) aus einem für die emittierte Strahlung transparenten Material besteht.

18. Bestrahlungsanordnung nach einem der Ansprüche 14 bis 17, **dadurch gekennzeichnet, daß** die Niederdruckentladungslampe (3) mit einer Befestigungsvorrichtung zur Aufnahme unterschiedlich geometrisch ausgeformter Verdrängungskörper (11) ausgebildet ist.

19. Bestrahlungsanordnung nach einem der Ansprüche 14 bis 18, **dadurch gekennzeichnet, daß** der Verdrängungskörper (11) unregelmäßig ausgeformt ist, so daß der Kanal (13) zwischen Hüllkörper (7) und Verdrängungskörper (11) entlang der Längsrichtung unterschiedliche Breiten aufweist.

20. Bestrahlungsanordnung nach einem der Ansprüche 11 bis 19, **dadurch gekennzeichnet, daß** die Leuchtstofffolie (5) in Form eines Wechselrahmens auf dem Hüllkörper (7) montiert ist.

21. Bestrahlungsanordnung nach Anspruch 20, **dadurch gekennzeichnet, daß** die unterschiedlich dotierten Folien (5) auf zu- und abführenden Walzen (6) aufgewickelt sind.

22. Bestrahlungsanordnung für therapeutische Zwecke nach einem der Ansprüche 11, 14, oder 15, **dadurch gekennzeichnet, daß** die zu behandelnde Körperpartie mit einer Leuchtstofffolie (5) nach einem der Ansprüche 1 bis 11 verbandähnlich umwickelt ist.

23. Verfahren zur Herstellung einer Leuchtstofffolie nach Anspruch 1, umfassend folgende Verfahrensschritte:
a) Mischen eines Hydroxylpolydiorganosiloxans mit einem Organohydrogensiloxan,
b) Zuführen von Leuchtstoffpartikeln und
c) Erzeugen einer chemischen Reaktion mittels eines Platinkatalysators bei Raumtemperatur.

24. Verfahren zur Herstellung einer Leuchtstofffolie nach Anspruch 23, **dadurch gekennzeichnet, daß** das Hydroxylpolydiorganosiloxan aus verschiedenen Polymeren mit einer Mindestviskosität von 1000 Centipoise bei 25°C besteht.

25. Verfahren zur Herstellung einer Leuchtstofffolie nach Anspruch 24, **dadurch gekennzeichnet, daß** das Hydroxylpolydiorganosiloxan als Hydroxylpolydimethylsiloxan, dessen Copolymeren, Phenylmethylsiloxan und/ oder Polymethyl -3, 3, 3-Trifluoropropylsiloxan ausgebildet ist.

26. Verfahren zur Herstellung einer Leuchtstofffolie nach Anspruch 23, 24 oder 25, **dadurch gekennzeichnet, daß** das Organohydrogensiloxan als Silikon mit mindestens 2- silikongebundenen Wasserstoffatomen pro Molekül ausgebildet ist

27. Verfahren zur Herstellung einer Leuchtstofffolie nach Anspruch 26, **dadurch gekennzeichnet, daß** das Organohydrogensiloxan aus Homopolymeren, Copolymeren oder deren Mischungen besteht.

28. Verfahren zur Herstellung einer Leuchtstofffolie nach einem der Ansprüche 23-27, **dadurch gekennzeichnet, daß** der Platinkatalysator aus Platinchlorid, Platinsalzen oder Chlorplatinsäure besteht.

29. Verfahren zur Herstellung einer Leuchtstofffolie nach Anspruch 28, **dadurch gekennzeichnet, daß** die Chlorplatinsäure als Hexahydrat oder in wasserfreier Form vorliegt.

## Claims

1. Fluorescent film, more especially for use with a low-pressure discharge lamp (3), the fluorescent film (5) being in the form of a silicone elastomer, in which the fluorescent particles are embedded,
**characterised in that**
the silicone elastomer is producible by the following method:
a) mixing an hydroxylpolydiorganosiloxane with an organohydrogensiloxane,
b) supplying fluorescent particles, and
c) producing a chemical reaction by means of a platinum catalyst at ambient temperature.

2. Fluorescent film according to claim 1, **characterised in that** the hydroxylpolydiorganosiloxane comprises various polymers having a minimum viscosity of 1000 centipoise at 25° C.

3. Fluorescent film according to claim 2, **characterised in that** the hydroxylpolydiorganosiloxane is in the form of hydroxylpolydimethylsiloxane, the copolymers thereof, phenylmethylsiloxane and/or polymethyl-3,3,3-trifluoropropylsiloxane.

4. Fluorescent film according to claim 2 or 3, **characterised in that** the organohydrogensiloxane is in the form of silicone having at least 2 silicone-combined hydrogen atoms per molecule.

5. Fluorescent film according to claim 4, **characterised in that** the organohydrogensiloxane comprises homopolymers, copolymers or mixtures thereof.

6. Fluorescent film according to one of the preceding claims, **characterised in that** the platinum catalyst comprises platinum chloride, platinum salts or chloroplatinic acid.

7. Fluorescent film according to claim 6, **characterised in that** the chloroplatinic acid is present as hexahydrate or in a water-free form.

8. Fluorescent film according to one of the preceding claims, **characterised in that** the thickness of the fluorescent film (5) is between 10 and 800 µm.

9. Fluorescent film according to one of the preceding claims, **characterised in that** the surface density of the fluorescent particles is 1-20 mg/cm².

10. Fluorescent film according to one of the preceding claims, **characterised in that** the particle size of the fluorescent particles is between 5 and 15 µm.

11. Irradiation arrangement, including a low-pressure discharge lamp, provided with a UVC-permeable enveloping body, in which contactable electrodes protrude from externally, and a fluorescent layer, **characterised in that** the fluorescent layer is in the form of a fluorescent film (5) according to claim 1, in which fluorescent particles are embedded.

12. Irradiation arrangement according to claim 11, **characterised in that** the fluorescent film (5) is mounted on the outside of the enveloping body (7).

13. Irradiation arrangement according to claim 12, **characterised in that** variably doped fluorescent films (5) are applied to the enveloping body (7).

14. Irradiation arrangement according to one of claims 11 to 13, **characterised in that** a displacement body (11) is disposed in the enveloping body (7), so that channels (13) are formed between the enveloping body (7) and the displacement body (11).

15. Irradiation arrangement according to claim 14, **characterised in that** the displacement body (11) is in the form of a closed hollow body.

16. Irradiation arrangement according to one of claims 14 or 15, **characterised in that** a reflector layer (12) is applied at least partially to the outside of the displacement body (11).

17. Irradiation arrangement according to one of claims 14 to 16, **characterised in that** the displacement body (11) is formed from a material which is transparent to the emitted radiation.

18. Irradiation arrangement according to one of claims 14 to 17, **characterised in that** the low-pressure discharge lamp (3) is provided with a mounting apparatus for the accommodation of displacement bodies (11) of different geometrical configurations.

19. Irradiation arrangement according to one of claims 14 to 18, **characterised in that** the displacement body (11) has an irregular configuration, so that the channel (13) between the enveloping body (7) and the displacement body (11) has different widths along the longitudinal direction.

20. Irradiation arrangement according to one of claims 11 to 19, **characterised in that** the fluorescent film (5), in the form of a changing frame, is mounted on the enveloping body (7).

21. Irradiation arrangement according to claim 20, **characterised in that** the variably doped films (5) are wound over supplying and discharging rollers (6).

22. Irradiation arrangement for therapeutic purposes according to one of claims 11, 14 or 15, **characterised in that** the part of the body to be treated is wound, in the form of a bandage, with a fluorescent film (5) according to one of claims 1 to 11.

23. Method of producing a fluorescent film according to claim 1, including the following method steps:
a) mixing an hydroxylpolydiorganosiloxanes with an organohydrogensiloxane,
b) supplying fluorescent particles, and
c) producing a chemical reaction by means of a platinum catalyst at ambient temperature.

24. Method of producing a fluorescent film according to claim 23, **characterised in that** the hydroxylpolydiorganosiloxane comprises various polymers having a minimum viscosity of 1000 centipoise at 25° C.

25. Method of producing a fluorescent film according to claim 24, **characterised in that** the hydroxylpolydiorganosiloxane is in the form of hydroxylpolydimethylsiloxane, copolymers thereof, phenylmethylsiloxane and/or polymethyl-3,3,3-trifluoropropylsiloxane.

26. Method of producing a fluorescent film according to claim 23, 24 or 25, **characterised in that** the organohydrogensiloxane is in the form of silicone having at least 2 silicone-combined hydrogen atoms per molecule.

27. Method of producing a fluorescent film according to claim 26, **characterised in that** the organohydrogensiloxane comprises homopolymers, copolymers or mixtures thereof.

28. Method of producing a fluorescent film according to one of claims 23-27, **characterised in that** the platinum catalyst comprises platinum chloride, platinum salts or chloroplatinic acid.

29. Method of producing a fluorescent film according to claim 28, **characterised in that** the chloroplatinic acid is present as hexahydrate or in a water-free form.

## Revendications

1. Film en matière luminescente pour l'utilisation avec une lampe à décharge à basse pression (3), moyennant quoi le film en matière luminescente (5) est configuré comme élastomère de silicone, dans lequel les particules de matière luminescente sont noyées,
**caractérisé en ce que**
l'élastomère de silicone peut être fabriqué moyennant le procédé suivant :
a) mélange d'un hydroxyl-polydiorgano-siloxane avec un organo-hydrogéno-siloxane,
b) addition de particules de matière luminescente et
c) production d'une réaction chimique au moyen d'un catalyseur à platine à la température ambiante.

2. Film en matière luminescente selon la revendication 1, **caractérisé en ce que** l'hydroxyl-polydiorgano-siloxane consiste en différents polymères d'une viscosité minimale de 1000 centipoises à 25 °C.

3. Film en matière luminescente selon la revendication 2, **caractérisé en ce que** l'hydroxyl-polydiorgano-siloxane est configuré comme hydroxyl-polydiméthyl-siloxane, ses copolymères, le phényl-méthyl-siloxane et / ou le poly(méthyl-3, 3, 3-trifluoropropyl-siloxane).

4. Film en matière luminescente selon la revendication 2 ou 3, **caractérisé en ce que** l'organo-hydrogéno-siloxane est configuré comme silicone à deux atomes d'hydrogène au moins liés au silicium par molécule.

5. Film en matière luminescente selon la revendication 4, **caractérisé en ce que** l'organo-hydrogéno-siloxane consiste en homopolymères, en copolymères ou en leurs mélanges.

6. Film en matière luminescente selon l'une quelconque des revendications précédentes, **caractérisé en ce que** le catalyseur à platine consiste en chlorure de platine, en sels de platine ou en acide chloroplatinique.

7. Film en matière luminescente selon la revendication 6, **caractérisé en ce que** l'acide chloroplatinique est présent sous forme d'hexahydrate ou sous forme anhydre.

8. Film en matière luminescente selon l'une quelconque des revendications précédentes, **caractérisé en ce que** le film en matière luminescente (5) a une épaisseur comprise entre 10 et 800 µm.

9. Film en matière luminescente selon l'une quelconque des revendications précédentes, **caractérisé en ce que** la densité surfacique des particules de matière luminescente est de 1 à 20 mg/cm².

10. Film en matière luminescente selon l'une quelconque des revendications précédentes, **caractérisé en ce que** la granulométrie des particules de matière luminescente est de 5 à 15 µm.

11. Dispositif d'irradiation, comprenant une lampe à décharge à basse pression avec un corps enveloppe transparent aux UVC, dans lequel saillent des électrodes pouvant être contactées de l'extérieur et une couche de matière luminescente,
**caractérisé en ce que**
la couche de matière luminescente est configurée comme film en matière luminescente (5) selon la revendication 1, dans lequel sont noyées des particules de matière luminescente.

12. Dispositif d'irradiation selon la revendication 11, **caractérisé en ce que** le film en matière luminescente (5) est appliqué à la face extérieure du corps enveloppe (7).

13. Dispositif d'irradiation selon la revendication 12, **caractérisé en ce que** sur le corps enveloppe (7) sont appliqués des films en matière luminescente (5) différemment dopés.

14. Dispositif d'irradiation selon l'une quelconque des revendications 11 à 13, **caractérisé en ce que** dans le corps enveloppe (7) est disposé un corps de refoulement (11), de telle sorte qu'entre le corps enveloppe (7) et le corps de refoulement (11), il se forme des canaux (13).

15. Dispositif d'irradiation selon la revendication 14, **caractérisé en ce que** le corps de refoulement (11) est configuré comme corps fermé.

16. Dispositif d'irradiation selon l'une des revendications 14 ou 15, **caractérisé en ce qu'**une surface de réflexion (12) est appliquée au moins en partie sur la face extérieure du corps de refoulement (11).

17. Dispositif d'irradiation selon l'une quelconque des revendications 14 à 16, **caractérisé en ce que** le corps de refoulement (11) consiste en un matériau transparent à la radiation émise.

18. Dispositif d'irradiation selon l'une quelconque des revendications 14 à 17, **caractérisé en ce que** la lampe à décharge à basse pression (3) est configurée avec un dispositif de fixation pour la réception de corps de refoulement (11) de différentes formes géométriques.

19. Dispositif d'irradiation selon l'une quelconque des revendications 14 à 18, **caractérisé en ce que** le corps de refoulement (11) est de forme irrégulière, de telle sorte que le canal (13) entre le corps enveloppe (7) et le corps de refoulement (11) présente différentes largeurs le long de la direction longitudinale.

20. Dispositif d'irradiation selon l'une quelconque des revendications 11 à 19, **caractérisé en ce que** le film en matière luminescente (5) est monté sur le corps enveloppe (7) sous la forme d'un cadre interchangeable.

21. Dispositif d'irradiation selon la revendication 20, **caractérisé en ce que** les films différemment dopés (5) sont bobinés sur des rouleaux d'alimentation et d'évacuation (6).

22. Dispositif d'irradiation à usage thérapeutique selon l'une des revendications 11, 14 ou 15, **caractérisé en ce que** la partie du corps à traiter est bandée à la manière d'un pansement avec un film en matière luminescente (5) selon une des revendications 1 à 11.

23. Procédé de fabrication d'un film en matière luminescente selon la revendication 1, comprenant les étapes de procédé suivantes :
a) mélange d'un hydroxyl-polydiorgano-siloxane avec un organo-hydrogéno-siloxane,
b) addition de particules de matière luminescente et
c) production d'une réaction chimique au moyen d'un catalyseur à platine à la température ambiante.

24. Procédé de fabrication d'un film en matière luminescente selon la revendication 23, **caractérisé en ce que** l'hydroxyl-polydiorgano-siloxane consiste en différents polymères d'une viscosité minimale de 1000 centipoises à 25 °C.

25. Procédé de fabrication d'un film en matière luminescente selon la revendication 24, **caractérisé en ce que** l'hydroxyl-polydiorgano-siloxane est configuré comme hydroxyl-polydiméthyl-siloxane, ses copolymères, le phényl-méthyl-siloxane et / ou le poly(méthyl-3, 3, 3-trifluoropropyl-siloxane).

26. Procédé de fabrication d'un film en matière luminescente selon l'une des revendications 23, 24 ou 25, **caractérisé en ce que** l'organo-hydrogénosiloxane est configuré comme silicone à deux atomes d'hydrogène au moins liés au silicium par molécule.

27. Procédé de fabrication d'un film en matière luminescente selon la revendication 26, **caractérisé en ce que** l'organo-hydrogéno-siloxane consiste en homopolymères, en copolymères ou en leurs mélanges.

28. Procédé de fabrication d'un film en matière luminescente selon l'une quelconque des revendications 23 à 27, **caractérisé en ce que** le catalyseur à platine consiste en chlorure de platine, en sels de platine ou en acide chloroplatinique.

29. Procédé de fabrication d'un film en matière luminescente selon la revendication 28, **caractérisé en ce que** l'acide chloroplatinique est présent sous forme de d'hexahydrate ou sous forme anhydre.
